Europäisches Patentamt

European Patent Office

Office européen des brevets

(11) Publication number: **0 260 757**

**A1**

## (12) EUROPEAN PATENT APPLICATION

(21) Application number: **87201745.4**

(22) Date of filing: **11.09.87**

(51) Int. Cl.⁴: **C12N 15/00 , A61K 37/64**

(30) Priority: **12.09.86 NL 8602307**

(43) Date of publication of application:
**23.03.88 Bulletin 88/12**

(84) Designated Contracting States:
**BE CH DE FR GB IT LI LU NL SE**

(71) Applicant: **Stichting Centraal Laboratorium van de Bloedtransfusiedienst van het Nederlandse Rode Kruis Plesmanlaan 125 NL-1066 CX Amsterdam(NL)**

(72) Inventor: **Pannekoek, Hans Stommeerweg 36 NL-1431 EW Aalsmeer(NL)**

(74) Representative: **Smulders, Theodorus A.H.J. et al Vereenigde Octrooibureaux Nieuwe Parklaan 107 NL-2587 BP 's-Gravenhage(NL)**

(54) Recombinant DNA molecule; a process for producing human endothelial plasminogen activator inhibitor (PAI) or a mutant thereof; pharmaceutical composition; transformed host cells.

(57) The invention is based on succesfully cloning and expressing the human endothelial plasminogen activator inhibitor, PAI. The invention provides recombinant DNA comprising a DNA sequence coding for PAI or mutants thereof; a process for producing PAI or mutants thereof by culturing transformed host cells; pharmaceutical compositions comprising PAI or mutants thereof obtained therein; and transformed host cells capable of expressing PAI or mutants thereof.

EP 0 260 757 A1

## Recombinant DNA molecule; a process for producing human endothelial plasminogen activator inhibitor (PAI) or a mutant thereof; pharmaceutical composition; transformed host cells.

This invention relates to a recombinant DNA molecule for use in cloning and/or expressing a DNA sequence in host cells. The invention also relates to a process for producing human endothelial plasminogen activator inhibitor or a mutant thereof, as well as to a pharmaceutical composition having an effect on blood coagulation or fibrinolysis, comprising human endothelial plasminogen activator inhibitor or a mutant thereof, and to transformed host cells.

Plasminogen activators (PA's) are serine proteases which play an important role in the vascular fibrinolysis and are also involved in several other physiological processes, such as ovulation, cell migration, epithelial differentiation and activation of "latent" collagenase. The regulation of the expression of PA's is therefore of essential importance for a normal body function. The enzyme considered to be primarily responsible for the lysis of fibrin clots is plasmine. Under normal physiological conditions, plasmine does not occur in the blood, but is formed from the proenzyme plasminogen upon activation by plasminogen activators, such as tissue-type plasminogen activator (t-PA) and urokinase (u-PA). A potential plasminogen activator source is the vascular wall endothelium. In addition plasminogen activators occur in other cell types and also in the blood.

Generally speaking, proteolytic systems are regulated by protease inhibitors. So is the fibrinolytical system. Fibrinolysis inhibitors can roughly be classified in two categories. Inhibitors, such as alpha-2-antiplasmine and alpha-2-macroglobuline, which are mainly active at the plasmine level and are found in plasma, and plasminogen activator inhibitors, which have been specifically demonstrated in tissues or isolated cell cultures (Levin and Loskutoff, 1979; Christensen et al., 1982; Holmberg et al., 1978; Emeis et al., 1983; Loskutoff et al., 1983; Crutchly et al., 1981). The structure and function of the first category of inhibitors has been described in detail. This has been done to a lesser extent with plasminogen activator inhibitors. Well-documented are the observations that fibroblasts and vascular endothelial cells produce inhibitors of u-PA (Levin and Loskutoff, 1979; Loskutoff et al., 1983; Crutchley et al., 1981; Dosne et al., 1978; Loskutoff and Edgington, 1981; Baker et al., 1980). The physiological significance of these inhibitors, however, is unclear. Recently it has been found that cultured endothelial cells also produce inhibitors of t-PA (Emeis et al., 1983; Van Mourik et al., 1984; Levin, 1983). Now, several observations raise the presumption that these vascular endothelial cell inhibitors play an important role in the regulation of fibrinolysis. In fact, in patients with an elevated thrombotic tendency it has been demonstrated that in some cases this condition is linked to an increased concentration of a circulating inhibitor (Chmielewska et al., 1983; Nilsson and Tengborn, 1984). This t-PA inhibitor (PAI) exhibits similarities to the vascular t-PA inhibitor (PAI) as regards molecular weight (about 50,000 daltons) and stability under denaturing conditions (Van Mourik et al., 1984; Levin, 1983; Nilsson and Tengborn, 1984; Wiman et al., 1984). It was also found that PAI carries the character of an acute-phase protein (Kruithof et al., 1985; Juhan-Vague et al., 1985) and can be made or secreted by endothelial cells in various clinical situations (inflammations, liver disorders, surgical operations). Possibly, this response leads to additional protection against local or systemic disorder of proteolytic systems. In view of the biological properties of PAI, this protease inhibitor is possibly of therapeutic interest. Not only in those clinical situations in which the hemostatic balance is disturbed (e.g. diffuse intravasal clotting, congenital clotting defects, such as hemophilia, trobocytopenia), and can be normalized by the administration of PAI as an antifibrinolytic but also in the case of thromboembolic conditions, in which the administration of t-PA is indicated. In the medical world there has been a wide interest for t-PA as an antithrombolytic since several years. This interest is based on the observation that t-PA specifically catalyses the decomposition of fibrin clots, and not, like streptokinase or u-PA the decomposition of circulating fibrinogen. The administration of these last two proteases not seldom leads to bleeding complications. These complications are observed to a lesser extent in a t-PA therapy. Experience has taught, however, that to effect an effective recirculation, high t-PA doses are required (Van de Werf et al., 1984). On the one hand, this is attributed to the rapid clearance of t-PA via the liver, and on the other hand, complex formation with PAI and, hence, inactivation of t-PA is held responsible for the rapid turnover of the t-PA administered. The cloning of genetic material coding for the human endothelial plasminogen activator inhibitor (PAI) and the expression of biologically active PAI in tissue culture cells, as will be described herein, opens the way towards the construction of molecular variants of PAI which do bind to t-PA, but do not deactivate t-PA. Such molecular variants can possibly be administered together with t-PA in a thrombolytic therapy, and may function as a competitive component for the plasma-circulating PAI (natural PAI). Such compositions of competitive PAI also offer potential uses in patients with thromboembolic conditions, in which it is observed that an elevated (endogenous) PAI level is responsible for the thrombotic tendency (Chmielewska et al., 1983; Nilsson and Tenborn, 1984; Kruithof et al., 1985; Juhan-Vague et al., 1985).

The inventors have now succeeded in cloning and expressing the human endothelial plasminogen activator inhibitor (PAI).

The invention is embodied in the first place in a recombinant DNA molecule for use in cloning and/or expressing a DNA sequence in host cells, which recombinant DNA molecule, in addition to a vector portion comprises a DNA sequence coding for human endothelial plasminogen activator inhibitor or a mutant thereof.

The invention is further embodied in a process for producing human endothelial plasminogen activator inhibitor or a mutant thereof, which comprises culturing, in a manner known per se, host cells which have been transformed using such a recombinant DNA molecule, and recovering the human endothelial plasminogen activator inhibitor or mutant thereof produced by the host cells.

The invention is also embodied in a pharmaceutical composition having an effect on blood coagulation or fibrinolysis, comprising human endothelial plasminogen activator inhibitor or a mutant thereof, produced using such a process, and in host cells, which, as a result of transformation by means of a recombinant DNA molecule as defined above, are capable of producing human endothelial plasminogen activator inhibitor or a mutant thereof.

By the words "a mutant thereof" are understood both natural mutants, such as allelic forms, and artificial mutants, in particular mutants whose properties are equal to, or even superior to, those of the cloned human endothelial plasminogen activator inhibitor, and mutants which do complex with t-PA, but do not prevent the biological activity of t-PA and/or delay the clearance of t-PA in the liver.

In case the words "a mutant thereof" relate to the DNA sequence for PAI, we also mean, in addition to the meanings mentioned above, deviating DNA sequences which, however, as a result of the "degeneration" of the genetic code yet code for the same amino acid sequence.

The term "host cells" as used herein means live organisms, such as bacteria, yeasts and fungi, as well as animal or human cell cultures.

It will be clear to those skilled in the art that the vector portion of the recombinant DNA molecules according to the invention must be selected on the basis of the kind of host cells in which the PAI or mutant thereof is desired to be cloned and/or expressed. Vectors suitable for the transformation of bacteria, yeasts, fungi and animal or human cell lines, mostly plasmids, are known per se to those skilled in the art. The term "transformation" is used here in the broad sense, and in addition to transformation in its actual sense, also comprises transduction (or transfection).

We will describe in detail hereinafter how the human endothelial plasminogen activator inhibitor has been cloned and expressed.

Starting from human endothelial polyA$^+$RNA, double-stranded copy-DNA (cDNA) was made in vitro, which by means of Sepharose CL-4B chromatography was size-fractionated. cDNA longer than about 600 base pairs (bp) was inserted into the expression vector pUC9 by means of G-C tailing.

The transformation of Escherichia coli DHI resulted in an expression library of about 60,000 colonies, about 90% of which carry recombinant DNA plasmids with a human endothelial cDNA insert having a length of at least 600 bp. The conies were analyzed for the presence of human endothelial plasminogen activator inhibitor (PAI) like polypeptides. For this purpose, a screening was used, employing a heterologous antiserum, i.e. rabbit anti-bovine PAI IgG, which antiserum is known to form immune complexes with the human endothelial PAI. As a second antibody, which in turn binds to the rabbit anti-bovine PAI IgG, $^{125}$I-labeled sheep anti-rabbit IgG was used. After autoradiography, three positive colonies were found, in duplicate, one positive colony of which was analyzed by means of restriction enzyme analysis on agarose gels, and by means of DNA sequencing techniques. It was concluded that the human cDNA insert with a length of about 2150 bp lacks a portion of the genetic information of the amino-terminal part of PAI. A second screening of the colony library with a synthetic oligonucleotide (24-mer), the nucleotide sequence of which is complementary to one of the DNA strands of the PAI cDNA insert (2150 bp) resulted in a number of colonies which were found to contain recombinant DNA plasmids with a cDNA insert of about 2350 bp. The genetic information located on the cDNA insert with a length of about 2350 bp codes for bioactive PAI, which could be detected after in-vivo synthesis in tissue culture cells transfected with a plasmid DNA carrying PAI cDNA with a length of about 2350 base pairs. The predicted amino acid sequence derived from the ascertained complete base sequence of the coding part of the 2350 bp fragment showed that there is a significant homology between PAI and other plasma protease inhibitors, such as antithrombin III, alpha-2-antiplasmin and alpha-1-antitrypsin. An Escherichia coli K12 DHI (pSV2/PAI2350 ex) strain containing the plasmid pSV2/PAI2350 ex described in the experimental section was deposited with the Centraalbureau voor Schimmelcultures (CBS) at Baarn, the Netherlands, on September 10, 1986 under number CBS 366.86.

Screening of the human endothelial cell expression library for the presence of antigen determinants related to PAI

A human endothelial cDNA library was constructed, using the Escherichia coli DHI strain as a host and the plasmid pUC9 as a vector (Viera and Messing, 1982). This plasmid carries the promotor of the lactose operon of E. coli and a short amino-terminal part of the first gene of the lactose operon, i.e., beta-galactosidase, and hence translation initiation elements. Fusion of cDNA, in the correct orientation and the correct translation reading frame of beta-galactosidase, leads to the synthesis of fusion proteins. The data that PAI is synthesized in human vascular endothelial cells and that therefore specific PAI mRNA synthesis will take place in these cells, justifies the supposition that PAI cDNA will also be formed during cDNA synthesis with total human endothelial polyA RNA. The cDNA library constructed as indicated in Materials and Methods consists of about 60,000 colonies which in principle all carry different recombinant DNA plasmids. About 90% of the colonies contain recombinant DNA plasmids, while about 10% of the colonies exclusively contain the vector pUC9. The colony library referred to is present on 13 nitrocellulose filters (diameter 13 cm) on which the colonies are uniformly spread. The colonies on the duplex filters were lysed and the proteins fixed on the filters as described (Verweij et al., 1985). By means of a heterologous antiserum, i.e. rabbit anti-bovine PAI IgG and subsequently with an anti-antibody ($^{125}$I-labelled sheep anti-rabbit IgG), a screening for the presence of potential PAI antigenic determinants was carried out. After autoradiography, three colonies were found which were radioactive on both duplicate filters, which is indicative of the presence of PAI antigenic determinants in the colonies concerned. These colonies were purified streaked pure and screening was repeated. Now, one "clone" was found to give a strong signal in the above detection method after autoradiography, while the other two clones gave a weaker signal. The clone giving the strong signal was selected for further analysis. From it, a recombinant DNA plasmid was isolated, which will be referred to as pPAI2150 hereinafter.

Restriction enzymes DNA sequence analysis of pPAI2150 DNA

The plasmid tentatively called pPAI2150 was isolated by a standard process (Maniatis et al., 1982). The digestion of this plasmid DNA with either HindIII or BamHI, or with both enzymes, and the analysis of the digestion patterns on a 0.8% agarose gel after electrophoresis showed that both after digestion with HindIII and after digestion with BamHI, a single fragment with a length of about 4800 bp is formed, whereas after digestion with both restriction enzymes two fragments are generated with a length of about 2675 bp and about 2150 bp, respectively. The 2675 bp fragment can be attributed to the vector pUC9, while the fragment of about 2150 bp potentially codes for PAI antigenic deter minant(s).

For determining a portion of the base sequence of the inserted cDNA fragment, the HindIII-BamHI fragment of pPAI2150, which possibly contains PAI cDNA, was isolated and then inserted into the double-stranded replication form of the E. coli-bacteriophage M13mp8 and thereafter the single-stranded DNA form of the recombinant DNA bacteriophage was isolated (Sanger et al., 1977). By means of the so-called dideoxy chain-termination procedure, the base sequence of a segment of the 2150 bp HindIII-BamHI fragment was determined with the universal sequence primer (New England Biolabs, Beverly, Mass.). This segment contains the 5' terminal part of the insert, which can be concluded from the known position of the unique HindIII, PstI and BamHI sites on the vector pUC9.

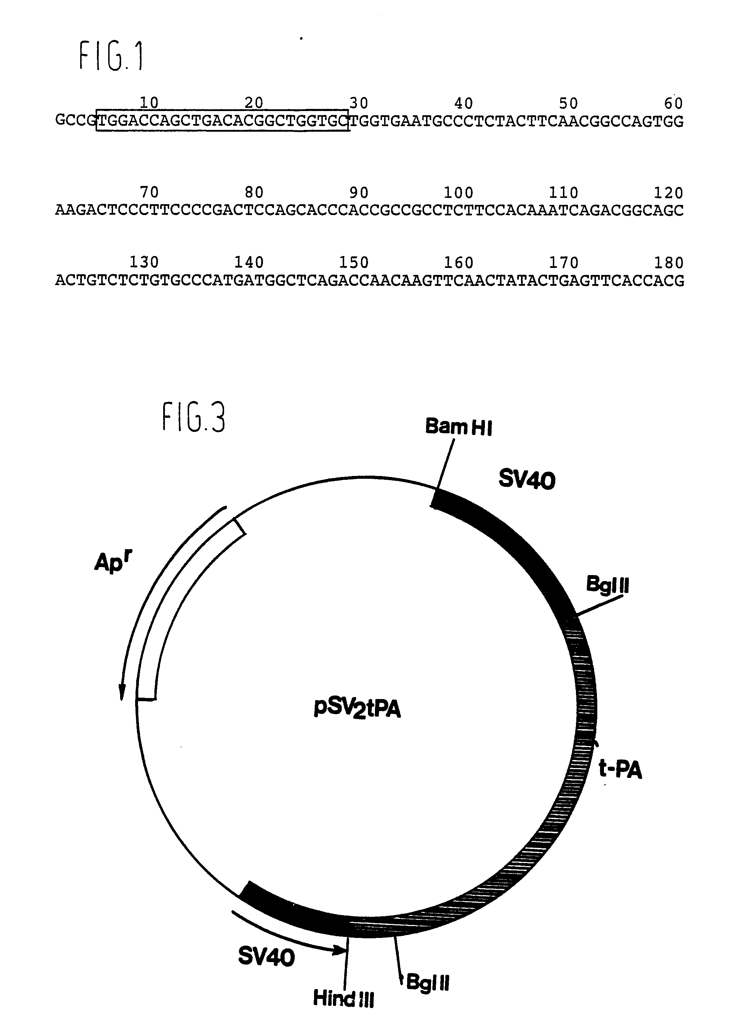

The base sequence is shown in Fig. 1. From this, the following conclusions can be drawn. Two of the three possible translation reading frames contain a plurality of stop codons and therefore cannot qualify as a potential reading frame of PAI. The remaining translation reading frame does not contain any stop codons in both directions. However, on the ground of these observations it cannot yet be concluded whether the 5'-terminal part of the cDNA contains an ATG codon functioning as a translation-initiation codon. In two ways it was subsequently tried to obtain an indication about the question whether the cDNA insert of about 2150 bp contains the complete genetic information for PAI. On the one hand, a Northern blotting experiment was conducted, in which total endothelial polyA RNA was size-fractionated by means of electrophoresis and then hybridized with radioactively labelled 2150 bp cDNA fragment to determine the length of PAI mRNA, and on the other hand a synthetic oligonucleotide was prepared, by means of which it can be determined whether the colony library contains clones with inserts longer than 2150 bp.

## Northern blotting of human endothelial polyA RNA

Total RNA and total polyA RNA were isolated from human endothelial cells and after denaturation in formamide-formaldehyde size-fractionated by means of electrophoresis on a formaldehyde-agarose gel. After blotting fractionated RNA on a nitrocellulose membrane filter, it was hybridized with HindIII-BamHI PAI2150 cDNA fragment, which after radioactive labelling with $^{32}$P by means of nick translation was denatured by boiling (Maniatis et al., 1982). As length markers, ribosomal RNA was used (28 and 18 S) and also total endothelial RNA, after electrophoresis, was hybridized with radiolabelled cDNA coding for tissue-type plasminogen activator (t-PA). This radioactive t-PA probe hybridizes with t-PA mRNA, which has a length of about 2540 nucleotides (Pennica et al., 1983). The results show that the radio-label led PAI2150 cDNA probe hybridizes with a human endothelial mRNA with a length of about 2300 nucleotides. These observations indicate that the genetic information potentially coding for PAI and located on the plasmid pPAI2150 probably lacks a (relatively short) segment at the 5′ side of the complete PAI cDNA. It was expected, therefore, that the clone in question lacks genetic information potentially coding for a number of amino-terminal amino acids of PAI. On the ground of this conclusion, a synthetic oligonucleotide was made, with a length of 24 nucleotides, which is complementary to nucleotides 5-28 of the sequence shown in Fig. 1. The rationale is for this oligonucleotide to be radio-labelled at the 5′ side with T4 polynucleotide kinase and gamma-labelled $^{32}$p ATP, and for this "probe" to be subsequently used for again screening the human endothelial cDNA expression library for other clones potentially containing PAI cDNA, which possibly carry more 5′ located genetic information but are not necessarily "expressed". In this arrangement, such clones will contain PAI cDNA which either has not the same orientation as has the amino-terminal segment of beta-galactosidase on the pUC9 vector, or has not the same translation reading frame.

## Screening of the human endothelial cDNA library with a synthetic oligonucleotide (24-mer)

On the ground of the specific base sequence - (about 250 bp) of the 5′ terminal part of the cDNA insert of the pPAI2150 plasmid (see Fig. 1), a 24-mer was prepared by means of a DNA synthesizer type 381A (Applied Biosystems, Foster City, Cal.), which is complementary to nucleotides 5-28. The sequence of the oligonucleotide is as follows: 5′ G C A C C A G C C G T G T C A G C T G G T C C A 3′. Screening of the human endothelial cell library with radio-labelled 24-mer resulted in 20 clones which on duplex filters after autoradiography were positive. Further identification of these 20 clones, which all contained recombinant DNA plasmids, was effected using standard techniques as described (Maniatis et al., 1982). The length of the cDNA inserts can again be determined after digestion with both Hin dIII and BamHI as, as explained before, it was found that these recombinant DNA plasmids also exhibited the feature that the cDNA inserts do not carry restriction sites for the restriction enzymes Hin dIII and BamHI. Characteristic of recombinant DNA plasmids having nucleotide homology with the pPAI2150 plasmid is the presence of a PvuII fragment with a length of about 550 bp. The PvuII restriction site situated at the 5′ side of the cDNA inserts coincides with the complementary sequence of the synthetic oligonucleotide (24-mer), i.e. 5′CAGCTG3′. After digestion of the potential 20 pPAI DNA compositions with the PvuII, followed by Southern blotting of denatured DNA on nitrocellulose membrane filters, they were hybridized with radio-labelled HindIII-BamHI fragment of pPA2150 DNA, the length of which is about 2150 bp. An essential observation is that all of the 20 digested DNA preparations contain a PvuII restriction fragment with a length of about 550 bp, which hybridizes with the above probe. On the ground of these results, the 20 plasmids can also be regarded as potential pPAI plasmids.

The pPAI plasmids which on the basis of digestion with both HindIII and BamHI carry a cDNA insert of about 2350 bp were selected for further study. Such pPAI2350 DNA preparations may contain the complete genetic information for human PAI.

## In-vitro synthesis of polypeptides coded by PAI cDNA

A recombinant DNA plasmid, called pSP65/PAI2350, was constructed as described in the section Materials and Methods. In-vitro transcription with the linear form of this plasmid (pSP65/PAI2350 DNA digested with HindIII) was carried out with the so-called SP6 RNA polymerase, which specifically initiates transcription at the SP6 promotor located at the 5′ side of the 2350 bp HindIII-BamHI PAI cDNA insert on pSP65/PAI2350 DNA. In this way, so-called "run off" transcripts were generated, having a length of about 2350 nucleotides. The resulting RNA was used as a matrix for in-vitro protein synthesis by means of a reticulocyte lysate. After in-vitro protein synthesis, the polypeptides formed were electrophoresed, by means of SDS-polyacrylamide gels. When de novo polypeptides were radio-labelled during the synthesis (with $^{35}$S-labelled methionine) it was found that the most dominant band was formed by a polypeptide with an apparent molecular weight of about 43,000 daltons. De novo polypeptides with a lower molecular weight may be the result of so-called internal translation initiations, using internal AUG codons in PAI mRNA. When no RNA is added to the protein synthesizing system, no polypeptide is produced with an (apparent) molecular weight of about 43,000 daltons. This experiment shows that the insert of about 2350 base pairs does not contain nonsense codons and therefore can code for a protein of the expected size. It has been demonstrated that the bovine PAI is a glyco-protein (Van Mourik et al., 1984). The difference in apparent molecular weight between the in-vitro synthesized product (43,000 daltons) and human endothelial PAI (52,000 daltons; Sprengers et al., 1984) can be attributed to carbohydrate chains on the PAI polypeptide.

### In-vivo synthesis of biologically PAI coded by PAI cDNA:

A recombinant DNA plasmid was constructed by means of a procedure as indicated in the section Materials and Methods. Essential elements present in this plasmid can be summarized as follows: the origin of replication, and the beta-lactamase gene of the E. coli plasmid pBR322, the "early" promotor, polyadenylation signal, splice signal of the eukaryotic virus SV40, and the part of the complete PAI2350 cDNA insert of the plasmid pPAI2350 located between the EcoRI restriction site 52 and the BglII restriction site 1477. Restriction enzyme analysis of the resulting plasmid, referred to hereinafter as pSV2/PAI2350 ex, showed that the PAI2350 cDNA fragment has been inserted in the correct orientation relative to the early promotor of SV40. The early promotor of SV40 serves in this construct as a transcription promotor for the genetic information potentially coding for PAI.

Transfection of Mouse Ltk cells with a purified pSV2/PAI2350 ex DNA preparation, followed by a period of five days for so-called transient expression of the genes located on the plasmid (Lopata et al., 1984) results in conditioned medium that can be tested for synthesized and secreted polypeptides.

For this purpose the conditioned medium was analyzed by means of the "reverse fibrin autoradiography" (RFA) technique as described (Loskutoff et al., 1983; also see Materials and Methods). From the results (not shown) a number of conclusions can be drawn. The PAI2350 cDNA part of the plasmid pSV2/PAI2350 ex carries the genetic information for PAI, which is apparent from a specific zone in the overlay, which is resistant to the lysis of fibrin caused by plasmin (generated from plasminogen by plasminogen activator). This activity is not expressed when RFA is carried with conditioned medium of tissue culture cells transfected with a control plasmid also based on pSV2.

Another conclusion that can be drawn from this experiment concerns the size of the product coded by pSV2/PAI2350 ex DNA. Comparison with a standard (a partially purified PAI preparation from conditioned medium of human vascular endothelium cells) shows that, using this SDS-polyacrylamide gel electrophoresis system, a product is detected having an identical (apparent) molecular weight. On the ground of this observation, the conclusion can be drawn that "native" PAI from conditioned medium of human endothelial cells has the same properties as the recombinant DNA product coded by PAI cDNA, constructed and isolated as indicated in the preceding paragraphs. The (apparent) molecular weight of the human endothelial plasminogen activator inhibitor (PAI) is about 52,000 daltons (Sprengers et al., 1984), which corresponds to the (apparent) molecular weight of the bovine endothelial inhibitor (PAI) (Van Mourik et al., 1984).

From these results, we conclude that the described cDNA fragment PAI2350 contains the genetic information coding for biologically active PAI.

### Base sequence of PAI cDNA and the predicted amino acid sequence of PAI

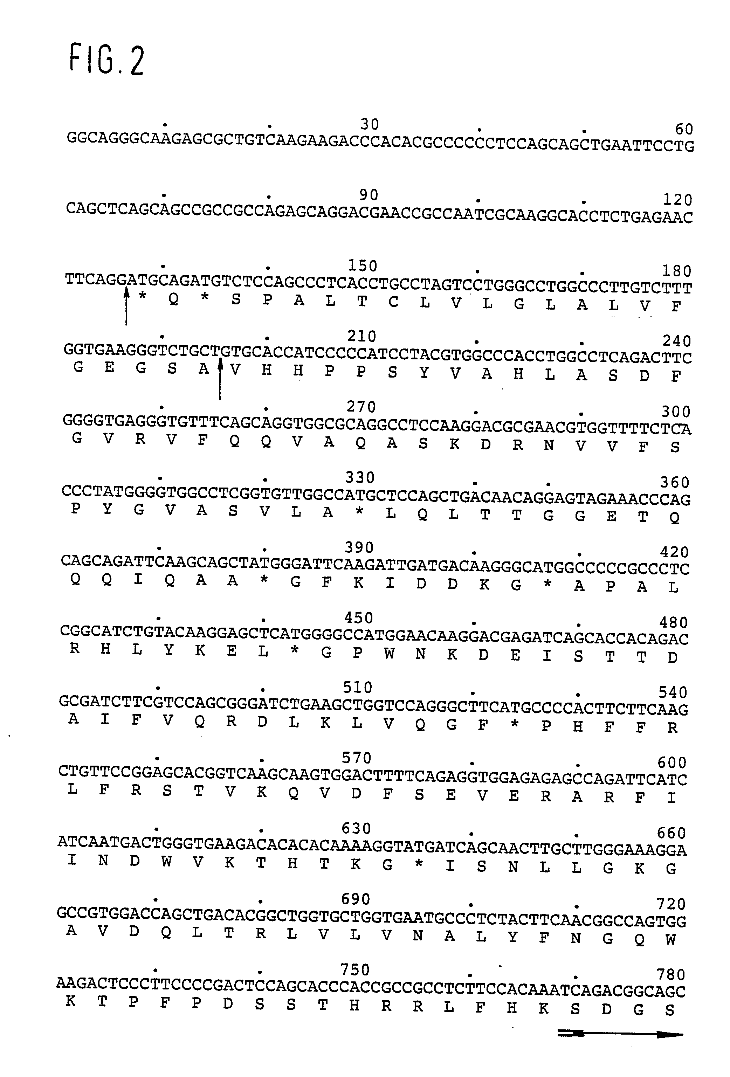

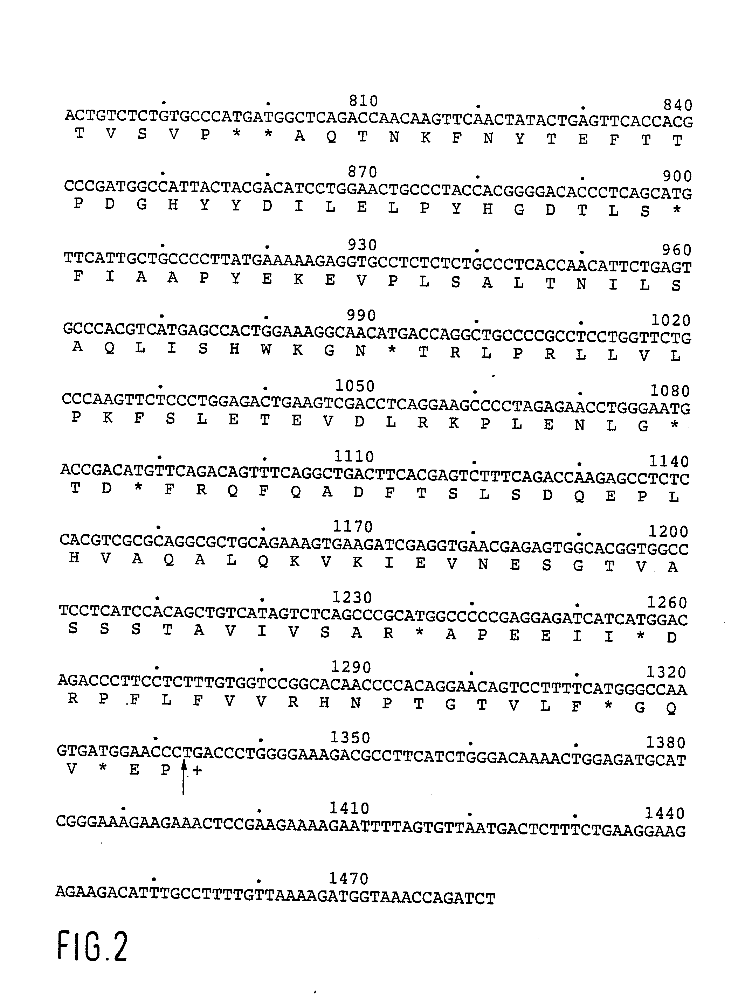

Both by means of the chemical degradation method (Maxam and Gilbert, 19877) and by means of the dideoxy method (Sanger et al., 1977), the nucleotide sequence of both DNA strands of PAI cDNA was determined. The results are shown in Fig. 2. The specific base sequence of PAI cDNA permits predicting the amino acid sequence of the PAI protein. A comparison of the amino acid sequence of PAI with other proteins shows that there is an extensive homology between PAI and other plasma protease inhibitors, i.e., antithrombin III, alpha-2-antiplasmin and alpha-1-antitrypsin. The finding that the human plasma protease inhibitors antithrombin III, alpha-2-antiplasmin, alpha-1-antitrypsin and ovalbumin show similarities in amino acid sequence has been reported before (Lijnen et al., 1982).

### Materials and methods

## DNA analysis

The isolation of plasmid DNA, the analysis of DNA digested with restriction enzymes by means of agarose and polyacrylamide gel electrophoresis, "Southern" and "Northern" blotting, radiolabelling of DNA, either by nick translation or by T4 polynucleotide kinase, and the technique of colony hybridisation were carried out as described (Maniatis et al., 1982). Determining the sequence of the bases in the DNA (DNA sequencing) was carried out both by the dideoxy method (Sanger et al., 1977) and by the chemical degradation method - (Maxam and Gilbert, 1977).

## Tissue cultures

Endothelial cells were isolated from human placental veins as described by Jaffe et al. (1973) with some modifications as indicated by Willems et al. (1982). Cells used for RNA isolation had undergone three or four passages and reached confluence before being harvested. The cells were harvested using trypsinization and then washed with 10 mM sodium phosphate (pH 7.4), 0.14 M NaCl and then frozen in liquid nitrogen.

## The isolation and characterization of human endothelial polyA RNA

The procedure for the isolation of human endothelial polyA RNA and the analysis for intactness of such preparations were effected as described before (Verweij et al., 1985).

## The purification of bovine endothelial plasminogen activator inhibitor

Bovine PAI was purified to homogeneity from serum-free conditioned medium of cultured bovine aortal endothelial cells as described before (Van Mourik et al., 1984). This purified preparation was used to immunize rabbits and the immunoglobulin G (IgG) fraction was subsequently purified by ammonium sulphate precipitations and DEAE-Sephadex chromatography. The heterologous anti-bovine PAI IgG preparation is capable of forming specific immune complexes with human endothelial PAI (Van Mourik, unpublished observation). This property of this antiserum makes it possible to determine the presence of human endothelial PAI.

## Copy DNA (cDNA) synthesis and the transformation of Escherichia coli DHI

The cDNA synthesis, in which total human endothelial polyA RNA was used as a substrate, was carried out following the procedure described by Gubler and Hoffman (1983). The resulting cDNA was size fractionated by means of Sepharose CL-4B chromatography, whereafter cDNA longer than about 600 base pairs (bp) was extended at both 3' ends with C residues, using the enzyme terminal transferase. This so-called "tailed" cDNA preparation was annealed with a twice molar excess of vector, i.e., a G-tailed pUC9 DNA (Viera and Messing, 1982). The transformation of strain Escherichia coli DHI (recA) with the recombinant DNA preparation obtained in the above manner was carried out according to the protocol described by Hanahan (1983).

## Testing for expression of PAI-like products in Escherichia coli DHI colonies

The method used was analogous to the procedure described before (Verweij et al., 1985). We note that both the rabbit anti-bovine PAI IgG and the "anti-antibodies" (i.e., $^{125}$I radio-labelled anti-rabbit IgG from a sheep immunized for the purpose) were preincubated for 2 hours at 4°C with a boiled lysate of E. coli DHI bacteria. For the detection of PAI antigen determinants which may potentially be present in the lysed colonies present on the nitrocellulose filters (Verweij et al., 1985), a 1 : 1,000 dilution of the rabbit anti-bovine PAI IgG preparation was used and thereafter in a second incubation about $2 \times 10^7$ counts per minute of radioactive sheep anti-rabbit IgG were used. After washing the filters (Verweij et al., 1985) autoradiography was performed for identifying "positive" colonies on duplex filters.

## In-vitro synthesis of PAI

An analysis with restriction enzymes of PAI cDNA inserted into the vector pUC9 showed that this cDNA does not contain restriction sites for, among others, the restriction enzymes HindIII and Bam HI. However, the vector has unique HindIII and BamHI sites on opposite sides of the PAI cDNA insert. Therefore, intact PAI cDNA inserts can be obtained after digestion of recombinant DNA plasmids with both HindIII and BamHI. The resulting plasmid pPAI2350, which is described in the following paragraphs, contains at the 5' side a unique BamHI site and at the 3' side of PAI cDNA a unique HindIII site. This BamHI-HindIII fragment, with a length of about 2350 bp was inserted into the plasmid pSP65, which had been previously digested also with BamHI and HindIII. The recombinant DNA plasmid pSP65/PAI2350 thus produced comprises a so-called SP6 promotor located immediately before the inserted PAI cDNA. This SP6 promotor functions as a specific starting point for RNA synthesis in-vitro, catalyzed by an RNA polymerase preparation coded by the Salmonella typhimurium bacteriophage SP6 (Melton et al., 1984). The digestion of pSP65/PAI2350 DNA with HindIII results in linear DNA, which can serve as a substrate for so-called "run off" transcription by means of the enzyme SP6 RNA polymerase and, among others, the four ribonucleotide triphosphates (ATP, GTP, CTP and UTP). The reactions were carried out as prescribed by the producer of the SP6 RNA polymerase preparation (New England Nuclear, Dreieich, Western Germany). About 0.1 µg PAI mRNA, made in vitro as described above, served as a matrix for in-vitro protein synthesis by means of a so-called reticulocyte lysate as described by the producer (Promega-Biotec, Wisconsin).

De novo polypeptides radio-labelled with $^{35}$S-methionine were analyzed by means of SDS-polyacrylamide gel electrophoresis (Laemmli, 1970).

## In-vivo synthesis of PAI

As a vector for transfection of Mouse Ltk cells, pSV2tPA was used (Mulligan and Berg, 1981; Van Zonneveld et al., 1985; see Fig. 3). This plasmid contains the origin of replication and the beta-lactamase gene of the plasmid pBR322 and in addition the so-called early promotor, a polyadenylation signal and a splice site of the eukaryotic virus SV40. In this vector, the complete coding t-PA cDNA is inserted between the unique HindIII and BgIII sites. The t-PA cDNA segment of the plasmid pSV2tPA was replaced by the PAI cDNA fragment with a length of about 2350 bp. For this purpose the plasmid pSV2tPA was digested with the enzyme HindIII and subsequently incubated with the enzyme DNA polymerase "large fragment" (New England Biolabs, Beverly, Mass.) and the four desoxyribonucleotide triphosphates (dATP, dGTP, dCTP, TTP), among others, to blunt the so-called sticky or cohesive ends (Maniatis et al., 1982). Thereafter the preparation was digested with BgIII. The plasmid pPAI2350 was digested with the enzyme EcoRI and subsequently, after blunting the sticky ends as described, this preparation was also digested with BgIII. Subsequently, by ligation with the enzyme T4 DNA ligase, the PAI cDNA fragment (1425 bp) was inserted into the vector pSV2 (Maniatis et al., 1982). The orientation of the PAI cDNA relative to the early promotor of SV40 could be determined after digestion of the recombinant DNA plasmids with resctriction enzymes en electrophoresis on agarose gels.

Transfection of Mouse Ltk cells with pSV2/PAI2350 ex DNA was carried out as described (Lopata et al., 1984). Five days after transfection, the conditioned medium was tested for the presence of PAI activity.

## Determination of PAI activity

The biological activity (inhibition PA activity) of in-vivo synthesized PAI was determined by so-called reverse fibrin autography, or RFA (Loskutoff et al., 1983). For this purpose, samples were electrophoresed in SDS-polyacrylamide gels and subsequently the gels were washed in a solution containing 2.5 % Triton-X-100 (Loskutoff et al., 1983). Such gels then obtained a solid fibrin overlay, formed by the addition of thrombin to a mixture of fibrinogen, agarose and plasminogen. The presence of small quantities of plasminogen activator caused the formation of plasmin in the entire overlay. The possible presence of PAI provides locally a zone that is resistant to lysis of fibrin by plasmin, because plasminogen activator forms an inactive complex with PAI, so that in this way no plasmin can be generated.

In the drawings, Fig. 1 shows the nucleotide sequence of the "upper" strand (direction 5' to 3') of the PAI2150 cDNA fragment.

The boxed portion is complementary to the synthetic 24-mer used to screen the colony library for the occurrence of recombinant DNA plasmids containing PAI cDNA. Fig. 2 shows the complete base sequence of the coding part of PAI2350 cDNA. The predicted amino acid sequence is shown in the one-letter code. x is the symbol for methionin (met); + is the symbol for a stop codon (in this case TGA); the arrows successively indicate: the translation initiation starting codon (ATG 127-129), the proposed site where the "ripe" PAI protein begins (Val) and the stop codon (TGA 1333-1335). Fig. 3 shows the plasmid pSV2t-PA used for the construction of the plasmid pSV2/PAI2350 ex, described in this application. The plasmid pSV2t-PA consists of 6333 base pair. The "black" part represents segments of the eukaryotic virus Simian Virus 40 (SV40) and in total covers 1350 base pairs. The "thin" line is from the Escherichia coli plasmid pBR322, contains the ampicillin-resistant gene and the origin of replication and covers 2883 base pairs. The hatched segment is from a recombinant DNA plasmid, a derivative of pBR322 containing the complete cDNA coding for tissue-type plasminogen activator (t-PA). The t-PA segment in pSV2t-PA located between the resctriction sites HindIII and BglII (hatched) comprises 2100 base pairs. After digestion of the plasmid pSV2t-PA with HindIII and Bgl II and purification of the fragment with a length of 4233 base pairs, the EcoRI-BglIII fragment containing PAI cDNA can be inserted. This gives the plasmid pSV2/PAI2350 ex.

References

Levin and Loskutoff, Thromb.Res. 15 (1979) 869-878.

Christensen et al., Thromb.Haemostasis 48 (1982) 24-26.

Holmberg et al., Biochim.Biophys. Acta 544 (1978) 128-137.

Emeis et al., Biochem.Biophys.Res.Commun. 110 (1983) 392-398.

Loskutoff et al., Proc.Natl.Acad.Sci. U.S.A. 80 (1983) 2956-2960.

Crutchley et al., Ann.N.Y. Acad.Sci. U.S.A. 370 (1981) 609-616.

Dosne et al., Thromb.Res.12 (1978) 377-387.

Loskutoff and Edgington, J. Biol.Chem. 256 (1981) 4142-4145.

Baker et al., Cell 21 (1980) 37-45.

Van Mourik et al., J. Biol.Chem. 259 (1984) 14914-14921.

Levin, Proc.Natl.Acad.Sci.U.S.A. 80 (1983) 8804-8808.

Chmielewska et al., Thromb.Res (1983) 427-436.

Nilsson and Tengborn, Haemostasis 14 (1984) 24 (abstract).

Kruithof et al., In: Progress in Fibrinolysis (Davidson, J.F.,Donati, M.B. and Coccheri, S.eds.). Vol. VII, Churchill Livingstone, Londen, pp. 130-132 (1985).

Juhan-Vague et al., In: Progress in Fibrinolysis (Davidson, J.F., Donati, M.B.and Coccheri, S.eds.). Vol. VII, Churchill Livingstone, Londen, pp. 146-149 (1985).

Wiman et al., J. Biol.Chem. 259 (1984) 3644-3647.

Van de Werf et al., New Engl.J.Med.310 (1984) 609-613.

Maniatis et al., Molecular Cloning Laboratory Manual, Cold Spring Harbor Laboratory, New York, (1982).

Sanger et al., Proc.Natl.Acad.Sci. U.S.A. 74 (1977) 5463-5467.

Maxam and Gilbert, Proc.Natl.Acad.Sci.U.S.A. 74 (1977) 560-564.

Jaffe et al., J. Clin.Invest.52 (1973) 2745-2756.

Willems et al., Exp.Cell. Res.139 (1982) 191-197.

Verweij et al., Nucleic Acids Res.13 (1985) 4699-4717.

Van Mourik et al., J. Biol.Chem. 259 (1984) 14914-14921.

Gubler and Hoffman, Gene 25(1983) 263-269.

Viera and Mesing, Gene 19(1982) 259-268.

Hanahan, J. Molec.Biol.166 (1983) 557-580.

Melton et al., Nucleic Acids Res.12 (1984) 7035-7056.

Laemmli, Nature 227 (1970) 680-685.

Mulligan and Berg, Proc.Natl.Acad.Sci.U.S.A. 78 (1981), 2072-2076.

Van Zonneveld et al, Proc.Natl.Acad.Sci. USA (1985, offered for publication)

Lopata et al., Nucleic Acids Res.12 (1984) 5707-5717.

Loskutoff et al., Proc.Natl.Acad.Sci.U.S.A. 80 (1983) 2956-2960.

Pennica et al., Nature 301 (1983) 214-221.

Sprengers et al., Biochim Biophys.Acta 801 (1984) 163-170.

Lijnen et al., Thromb.Haemostasis 48 (1982) 311-314.

## Claims

1. Recombinant DNA molecule for use in cloning and/or expressing a DNA sequence in host cells, which recombinant DNA molecule, in addition to a vector portion, contains a DNA sequence coding for human endothelial plasminogen activator inhibitor or a mutant thereof.

2. Recombinant DNA molecule as claimed in claim 1, comprising the DNA sequence shown in Fig. 2, or a mutant thereof.

3. A process for producing human endothelial plasminogen activator inhibitor or a mutant thereof, which comprises culturing host cells, in a manner known per se, said host cells being transformed using a recombinant DNA molecule as defined in claim 1 or 2, and recovering the human endothelial plasminogen activator inhibitor or mutant thereof, produced by the host cells.

4. Pharmaceutical composition having an effect on blood coagulation or fibrinolysis, comprising human endothelial plasminogen activator inhibitor or a mutant thereof, produced by the process as claimed in claim 3.

5. Host cells which as result of transformation by means of a recombinant DNA molecule as defined in claim 1 or 2 are capable of producing human endothelial plasminogen activator inhibitor or a mutant thereof.

# FIG.1

GCCG[TGGACCAGCTGACACGGCTGGTGC]TGGTGAATGCCCTCTACTTCAACGGCCAGTGG

AAGACTCCCTTCCCCGACTCCAGCACCCACCGCCGCCTCTTCCACAAATCAGACGGCAGC

ACTGTCTCTGTGCCCATGATGGCTCAGACCAACAAGTTCAACTATACTGAGTTCACCACG

# FIG.3

pSV2tPA

# FIG.2

```
                              30                            60
GGCAGGGCAAGAGCGCTGTCAAGAAGACCCACACGCCCCCCTCCAGCAGCTGAATTCCTG

                              90                           120
CAGCTCAGCAGCCGCCGCCAGAGCAGGACGAACCGCCAATCGCAAGGCACCTCTGAGAAC

                             150                           180
TTCAGGATGCAGATGTCTCCAGCCCTCACCTGCCTAGTCCTGGGCCTGGCCCTTGTCTTT
     * Q * S P A L T C L V L G L A L V F

                             210                           240
GGTGAAGGGTCTGCTGTGCACCATCCCCCATCCTACGTGGCCCACCTGGCCTCAGACTTC
 G E G S A V H H P P S Y V A H L A S D F

                             270                           300
GGGGTGAGGGTGTTTCAGCAGGTGGCGCAGGCCTCCAAGGACGCGAACGTGGTTTTCTCA
 G V R V F Q Q V A Q A S K D R N V V F S

                             330                           360
CCCTATGGGGTGGCCTCGGTGTTGGCCATGCTCCAGCTGACAACAGGAGTAGAAACCCAG
 P Y G V A S V L A * L Q L T T G G E T Q

                             390                           420
CAGCAGATTCAAGCAGCTATGGGATTCAAGATTGATGACAAGGGCATGGCCCCCGCCCTC
 Q Q I Q A A * G F K I D D K G * A P A L

                             450                           480
CGGCATCTGTACAAGGAGCTCATGGGGCCATGGAACAAGGACGAGATCAGCACCACAGAC
 R H L Y K E L * G P W N K D E I S T T D

                             510                           540
GCGATCTTCGTCCAGCGGGATCTGAAGCTGGTCCAGGGCTTCATGCCCCACTTCTTCAAG
 A I F V Q R D L K L V Q G F * P H F F R

                             570                           600
CTGTTCCGGAGCACGGTCAAGCAAGTGGACTTTTCAGAGGTGGAGAGAGCCAGATTCATC
 L F R S T V K Q V D F S E V E R A R F I

                             630                           660
ATCAATGACTGGGTGAAGACACACACAAAAGGTATGATCAGCAACTTGCTTGGGAAAGGA
 I N D W V K T H T K G * I S N L L G K G

                             690                           720
GCCGTGGACCAGCTGACACGGCTGGTGCTGGTGAATGCCCTCTACTTCAACGGCCAGTGG
 A V D Q L T R L V L V N A L Y F N G Q W

                             750                           780
AAGACTCCCTTCCCCGACTCCAGCACCCACCGCCGCCTCTTCCACAAATCAGACGGCAGC
 K T P F P D S S T H R R L F H K S D G S
```

```
                          810                             840
ACTGTCTCTGTGCCCATGATGGCTCAGACCAACAAGTTCAACTATACTGAGTTCACCACG
  T  V  S  V  P  *  *  A  Q  T  N  K  F  N  Y  T  E  F  T  T

                          870                             900
CCCGATGGCCATTACTACGACATCCTGGAACTGCCCTACCACGGGGACACCCTCAGCATG
  P  D  G  H  Y  Y  D  I  L  E  L  P  Y  H  G  D  T  L  S  *

                          930                             960
TTCATTGCTGCCCCTTATGAAAAAGAGGTGCCTCTCTCTGCCCTCACCAACATTCTGAGT
  F  I  A  A  P  Y  E  K  E  V  P  L  S  A  L  T  N  I  L  S

                          990                            1020
GCCCACGTCATGAGCCACTGGAAAGGCAACATGACCAGGCTGCCCCGCCTCCTGGTTCTG
  A  Q  L  I  S  H  W  K  G  N  *  T  R  L  P  R  L  L  V  L

                         1050                            1080
CCCAAGTTCTCCCTGGAGACTGAAGTCGACCTCAGGAAGCCCCTAGAGAACCTGGGAATG
  P  K  F  S  L  E  T  E  V  D  L  R  K  P  L  E  N  L  G  *

                         1110                            1140
ACCGACATGTTCAGACAGTTTCAGGCTGACTTCACGAGTCTTTCAGACCAAGAGCCTCTC
  T  D  *  F  R  Q  F  Q  A  D  F  T  S  L  S  D  Q  E  P  L

                         1170                            1200
CACGTCGCGCAGGCGCTGCAGAAAGTGAAGATCGAGGTGAACGAGAGTGGCACGGTGGCC
  H  V  A  Q  A  L  Q  K  V  K  I  E  V  N  E  S  G  T  V  A

                         1230                            1260
TCCTCATCCACAGCTGTCATAGTCTCAGCCCGCATGGCCCCCGAGGAGATCATCATGGAC
  S  S  S  T  A  V  I  V  S  A  R  *  A  P  E  E  I  I  *  D

                         1290                            1320
AGACCCTTCCTCTTTGTGGTCCGGCACAACCCCACAGGAACAGTCCTTTTCATGGGCCAA
  R  P  F  L  F  V  V  R  H  N  P  T  G  T  V  L  F  *  G  Q

                         1350                            1380
GTGATGGAACCCTGACCCTGGGGAAGACGCCTTCATCTGGGACAAAACTGGAGATGCAT
  V  *  E  P↑ +

                         1410                            1440
CGGGAAGAAGAAACTCCGAAGAAAGAATTTTAGTGTTAATGACTCTTTCTGAAGGAAG

                         1470
AGAAGACATTTGCCTTTTGTTAAAAGATGGTAAACCAGATCT
```

# FIG.2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | BIOCHIMICA ET BIOPHYSICA ACTA, vol. 801, 1984, pages 163-170, Elsevier, NL; E.D. SPRENGERS et al.: "Evidence for the presence of two different fibrinolytic inhibitors in human endothelial cell conditioned medium" * Whole document * --- | 1-5 | C 12 N 15/00 A 61 K 37/64 |
| Y | NUCLEIC ACIDS RESEARCH, vol. 13, no. 13, 1985, pages 4699-4717, IRL Press Ltd, Oxford, GB; C.L. VERWEY et al.: "Construction of cDNA coding for human von Willebrand factor using antibody probes for colony-screening and mapping of the chromosomal gene" * Whole document * --- | 1-3,5 | |
| Y | NATURE, vol. 277, 11th January 1979, Macmillan Journals Ltd, Basingstoke, GB; R.C. MULLIGAN et al.: "Synthesis of rabbit beta-globin in cultured monkey kidney cells following infection with a SV40 beta-globin recombinant genome" * Whole document * --- | 1-3,5 | TECHNICAL FIELDS SEARCHED (Int. Cl.4) C 12 N A 61 K |
| Y | NUCLEIC ACIDS RESEARCH, vol. 11, no. 20, October 1983, pages 7119-7136, IRL Press Ltd, Oxford, GB; R. BREATHNACH et al.: "Plasmids for the cloning and expression of full-length double-stranded cDNAs under control of the SV40 early or late gene promotor" * Whole document * --- -/- | 1-3,5 | |

The present search report has been drawn up for all claims

| Place of search | Date of completion of the search | Examiner |
|---|---|---|
| THE HAGUE | 11-12-1987 | DESCAMPS J.A. |

European Patent Office

EUROPEAN SEARCH REPORT

Application Number

EP 87 20 1745

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | THROMBOSIS RESEARCH, vol. 41, 1986, pages 151-160, Pergamon Press Ltd, US; M. SAWDEY et al.: "Messenger RNA for plasminogen activator inhibitor" * Whole document * | 1-3,5 | |
| Y | THROMBOSIS RESEARCH, vol. 31, 1983, pages 427-436, Pergamon Press Ltd, US; J. CHMIELEWSKA et al.: "Evidence for a rapid inhibitor to tissue plasminogen activator in plasma" * Whole document * | 4 | |
| P,X | THE EMBO JOURNAL, vol. 5, no. 10, 1986, pages 2539-2544; H. PANNEKOEK et al.: "Endothelial plasminogen activator inhibitor (PAI): a new member of the Serpin gene family" * Whole document * | 1-5 | |
| P,X | PROCEEDINGS OF THE NATIONAL ACADEMY OF SCIENCES OF THE USA, vol. 83, no. 18, pages 6776-6780, Washington, D.C., US; T. NY et al.: "Cloning and sequence of a cDNA coding for the human beta-migrating endothelial-cell-type plasminogen activator inhibitor" * Whole document * | 1-5 | TECHNICAL FIELDS SEARCHED (Int. Cl.4) |

The present search report has been drawn up for all claims

| Place of search | Date of completion of the search | Examiner |
|---|---|---|
| THE HAGUE | 11-12-1987 | DESCAMPS J.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)